# EUROPÄISCHE PATENTSCHRIFT

(11) **EP 2 205 767 B1**
(45) Veröffentlichungstag und Bekanntmachung des Hinweises auf die Patenterteilung: **27.07.2016**
(21) Anmeldenummer: 08843855.1
(22) Anmeldetag: 31.10.2008
(51) Int. Cl.: C12N 15/63, C12N 15/67, C12Q 1/68

(54) **VERFAHREN ZUR FESTSTELLUNG VON FRAMESHIFT-MUTATIONEN IN KODIERENDEN NUKLEINSÄUREN**
METHOD FOR DETERMINING FRAMESHIFT MUTATIONS IN CODING NUCLEIC ACIDS
PROCÉDÉ DE DÉTERMINATION DE MUTATIONS PAR DÉCALAGE DU CADRE DE LECTURE DANS DES ACIDES NUCLÉIQUES CODANTS

(30) Priorität: 02.11.2007 DE 102007052344
(43) Veröffentlichungstag der Anmeldung: 14.07.2010
(73) Patentinhaber: GENEART AG, 93053 Regensburg (DE)
(72) Erfinder: LISS, Michael, 93059 Regensburg (DE); DERER, Jutta, 93047 Regensburg (DE)
(74) Vertreter: Wöhler, Christian
(86) Internationale Anmeldenummer: PCT/EP2008/009223
(87) Internationale Veröffentlichungsnummer: WO 2009/056343

(56) Entgegenhaltungen:
- EP-A- 0 872 560
- RAK B ET AL: "INSERTION ELEMENT IS-5 CONTAINS A 3RD GENE" EMBO (EUROPEAN MOLECULAR BIOLOGY ORGANIZATION) JOURNAL, Bd. 3, Nr. 4, 1984, Seiten 807-812, XP002520783 ISSN: 0261-4189
- KUMAMOTO C A ET AL: "Characterization of the Escherichia coli protein-export gene secB" GENE, ELSEVIER, AMSTERDAM, NL, Bd. 75, Nr. 1, 30. Januar 1989 (1989-01-30), Seiten 167-175, XP023544380 ISSN: 0378-1119 [gefunden am 1989-01-30]
- MERINO ENRIQUE ET AL: "Antisense overlapping open reading frames in genes from bacteria to humans" NUCLEIC ACIDS RESEARCH, Bd. 22, Nr. 10, 1994, Seiten 1903-1908, XP002520784 ISSN: 0305-1048

## Beschreibung

Die vorliegende Erfindung betrifft ein Verfahren zur Bestimmung von Frameshift-Mutationen in kodierenden Nukleinsäuresequenzen.

Genetische Information ist in kodierenden Nukleinsäuren in Form von Basentriplets oder Kodons organisiert. Bei der Gensynthese treten jedoch häufig Fehler in Form unterschiedlicher Arten von Mutationen auf. Bei den Mutationen handelt es sich großteils um Einzelbasendeletionen und Insertionen (ca. 90 %). Im Gegensatz zu den meisten Substitutionen führen nahezu alle Insertionen und Deletionen zu so genannten Frameshifts. Geht eine Base eines Gens im Rahmen einer Mutation verloren (Deletion) oder wird eine Base hinzugefügt (Insertion), so verändert sich das Leseraster der nachfolgenden Basentriplets. Eine Frameshift-Mutation hat biologisch wesentlich größere Auswirkungen als eine Substitution. Ab der Insertion/ Deletion werden andere Aminosäuren kodiert und es kommt meist zu einem Abbruch der Translation durch out-of-frame Stoppkodons.

Mit zunehmender Länge eines synthetisierten Gens steigt die Wahrscheinlichkeit für das Auftreten einer Mutation stark an. Der Großteil der ungewollten Mutationen kommt durch das Fehlen oder die Insertion von Einzelbasen in Oligonukleotiden zustande. Die Wahrscheinlichkeit einer Substitution einer Base liegt bei der Synthese von Genen oder Genbibliotheken bei etwa 0,1-0,2 %o pro Position, die Wahrscheinlichkeit für eine Insertion/Deletion liegt bei 1,0-1,5 ‰ pro Position. Beispielsweise beobachteten Kong et al., Nucleic Acids Res. (2007), 35:e61 bei der Chipbasierten Gensynthese eine Deletionsrate von 1,48 ‰ pro Position und eine Substitutionsrate von 0,3 ‰ pro Position. Etwa die Hälfte der Substitutionen kommt wiederum durch PCR-Fehler zustande.

Die bei Insertionen und Deletionen auftretende Verschiebung des Leserasters kann genutzt werden, um über einen Reportervektor solche Nukleinsäure-Fragmente zu selektieren, deren Leseraster intakt ist. So offenbart die europäische Patentanmeldung EP 0 872 560 ein Verfahren zur Bestimmung von Frameshift-Mutationen, bei dem mittels homologer Rekombination ein Konstrukt erzeugt wird, das einen Promotor, ein zu untersuchendes Gen und im gleichen Leseraster mit dem zu untersuchenden Gen ein Reportergen enthält. Bei der Expression des Leserasters wird ein Fusionsprotein erhalten, welches die Reportergenfunktion beinhaltet. Dieses kann über phänotypische Eigenschaften nachgewiesen werden.

Problematisch ist allerdings, dass das zu untersuchende Gen für die in EP 0 872 560 beschriebene Vorgehensweise keine internen Stoppkodons innerhalb des offenen Leserasters (ORF) aufweisen darf. Dies ist jedoch bei vielen synthetisierten Genen der Fall.

Angesichts der obigen Problematik war die Aufgabe der vorliegenden Erfindung, ein Verfahren zur Bestimmung von Frameshift-Mutationen bereitzustellen, das für beliebige codierende Nukleinsäuren und insbesondere auch für Nukleinsäuren, welche interne Stoppkodons umfassen, geeignet ist.

Erfindungsgemäß wird diese Aufgabe gelöst durch ein Verfahren zur Bestimmung von Frameshift-Mutationen in kodierenden Zielnukleinsäuren, welche die Schritte umfasst:
(i) Bereitstellen einer Wirtszelle, umfassend eine doppelsträngige Nukleinsäure, welche eine kodierende Zielnukleinsäure und eine dazu komplementäre, kodierende Gegenstrangnukleinsäure umfasst, worin die Gegenstrangnukleinsäure in operativer Verknüpfung mit einem Reportergen in 3' Position vorliegt, und worin die Gegenstrangnukleinsäure so optimiert wird, dass sie keine Stopp-Kodons umfasst;
(ii) Bewirken der Expression der Gegenstrangnukleinsäure; und
(iii) Bestimmen, ob in der Wirtszelle eine Expression des Reportergens erfolgt, worin die Expression des Reportergens anzeigt, dass die Zielnukleinsäure keine Frameshift-Mutation aufweist,
wobei die Wirtszelle keine menschliche embryonale Zelle ist.

Der Begriff "keine Frameshift-Mutation" ist erfindungsgemäß so zu verstehen, dass die Zielnukleinsäure entweder überhaupt keine Frameshift-Mutation aufweist oder dass in der Zielnukleinsäure mehrere Mutationen vorliegen, die für sich betrachtet, zu einer Verschiebung des Leserahmens führen würden, die sich aber gegenseitig wieder aufheben. Beispielsweise kann in der Zielnukleinsäure eine Kombination von Insertionen und Deletionen vorliegen, die sich aber gegenseitig wieder aufheben, so dass keine Änderung des Leserasters auftritt und das Reportergen weiter korrekt abgelesen werden kann.

Die Erfinder haben herausgefunden, dass für die Bestimmung von Frameshift-Mutationen anstelle der kodierenden Zielnukleinsäure eine dazu komplementäre kodierende Gegenstrangnukleinsäure genutzt werden kann.

Der Begriff "kodierend" ist in diesem Zusammenhang so zu verstehen, dass die Gegenstrangnukleinsäure so beschaffen ist, dass sie die Expression des 3' verknüpften Reportergens ermöglicht. Die Gegenstrangnukleinsäure muss zu diesem Zweck aber nicht unbedingt durch einen offenen Leserahmen definiert sein.

Bei der Durchführung des erfindungsgemäßen Verfahrens wird zunächst eine Wirtszelle bereitgestellt, die eine doppelsträngige Nukleinsäure umfasst, welche eine kodierende Zielnukleinsäure und eine dazu komplementäre, kodierende Gegenstrangnukleinsäure enthält. Bei der Zielnukleinsäure handelt es sich vorzugsweise um eine synthetisch erzeugte Sequenz. Die Gegenstrangnukleinsäure ist in der Wirtszelle operativ mit einem Reportergen verknüpft, welches sich stromabwärts in 3'-Position befindet.

Wenn die Gegenstrangnukleinsäure intakt ist, d.h. keine Mutationen wie insbesondere Insertionen oder Deletionen aufweist, die eine Änderung des Leserasters zur Folge haben, so wird bei der Expression ein Fusionspolypeptid erhalten, das die von der Gegenstrangnukleinsäure kodierte Aminosäuresequenz sowie C-terminal davon die Aminosäuresequenz des von dem Reportergen kodierten Produkts aufweist. Wenn die Gegenstrangnukleinsäure dagegen eine Frameshift-Mutation aufweist, d.h. insbesondere eine Insertion oder Deletion, durch die sich das Leseraster ändert, so führt diese Verschiebung des Leserasters dazu, dass das Reportergen nicht in-frame zu der kodierenden Gegenstrangnukleinsäure liegt. Folglich wird das Reportergen nicht exprimiert. Der Reporter wird also nur dann exprimiert, wenn das Leseraster des Gegenstrangs intakt ist.

Wenn die Gegenstrangnukleinsäure keine Frameshift-Mutationen aufweist, so kann daraus geschlossen werden, dass auch die komplementäre Zielnukleinsäure selbst intakt ist und keine Frameshift-Mutationen aufweist.

Das Verfahren der vorliegenden Erfindung hat den Vorteil, dass selbst bei Vorliegen von internen Stoppkodons in der Zielnukleinsäure eine Bestimmung von Frameshift-Mutationen mittels Verwendung eines Reportergens möglich ist. Interne Stoppkodons der Zielnukleinsäure werden bei der Nutzung des Leserasters im Gegenstrang als Leu bzw. Ser translatiert. Die Nutzung eines Leserasters im Gegenstrang hat weiterhin den Vorteil, dass eine mögliche Toxizität des von der eigentlichen Zielnukleinsäure kodierten Proteins für die Wirtszelle nicht relevant ist, da nur der Gegenstrang translatiert wird.

Für die Durchführung des erfindungsgemäßen Verfahrens ist eine kodierende Gegenstrangnukleinsäure erforderlich. Ein Leseraster im Gegenstrang kann ggf. durch entsprechende Optimierung der Gegenstrangsequenz erreicht werden. Weiterhin sollte die Gegenstrangnukleinsäure keine internen Stoppkodons enthalten. Die komplementären Kodons zu den drei Stoppkodons sind relativ selten, daher enthält ein Leseraster im Gegenstrang in der Regel keine Stoppkodons. In einer bevorzugten Ausführungsform wird die Gegenstrangnukleinsäure ggf. so optimiert, dass keine internen Stoppkodons enthalten sind.

In einer bevorzugten erfindungsgemäßen Ausführungsform ist die Gegenstrangnukleinsäure in der Wirtszelle in 3'-Position operativ mit einem Reportergen verknüpft und in 5'-Position operativ mit einer Expressionskontrollsequenz verknüpft.

Eine Expressionskontrollsequenz ist erfindungsgemäß eine Nukleinsäuresequenz, welche die Transkription und Translation kontrolliert und reguliert. Die Expressionskontrollsequenz kann in der Wirtszelle konstitutiv oder regulierbar aktiv sein. Die Formulierung "operativ verknüpft" schließt ein geeignetes Startsignal (z.B. ATG oder AUG) vor der zu exprimierenden Nukleinsäuresequenz und die Beibehaltung des korrekten Leserasters ein, sodass die Expression der Nukleinsäuresequenz unter der Kontrolle der Expressionskontrollsequenz und die Erzeugung des von der Nukleinsäuresequenz kodierten Produktes ermöglicht wird. Wenn eine Nukleinsäure kein geeignetes Startsignal enthält, so kann ein solches Startsignal vor der Nukleinsäure eingefügt werden.

Die Bereitstellung einer Wirtszelle in Schritt (i) des erfindungsgemäßen Verfahrens beinhaltet in einer Ausführungsform das Einbringen eines Expressionsvektors in eine Wirtszelle, wobei der Expressionsvektor die doppelsträngige Nukleinsäure umfasst, welche eine kodierende Zielnukleinsäure und eine dazu komplementäre kodierende Gegenstrangnukleinsäure umfasst.

Expressionsvektoren sind dem Fachmann auf dem Gebiet der Molekularbiologie bekannt und beispielsweise in Sambrook et al., Molecular Cloning, A Laboratory Manual (1989), Cold Spring Harbour, Laboratory Press, beschrieben. Als Expressionsvektor kann beispielsweise ein Plasmid oder ein viraler Vektor verwendet werden.

Die Gegenstrangnukleinsäure kann in dem Expressionsvektor in einer Ausführungsform in operativer Verknüpfung mit einem Reportergen in 3'-Position vorliegen. Alternativ kann der Expressionsvektor in einer anderen Ausführungsform zusammen mit einem Reportervektor, welcher das Reportergen umfasst, in eine Wirtszelle eingebracht werden. In einer weiteren Ausführungsform kann der Expressionsvektor auch in eine Wirtszelle eingebracht werden, die bereits ein Reportergen enthält.

Vorzugsweise ist die Gegenstrangnukleinsäure in dem Expressionsvektor operativ mit einer Expressionskontrollsequenz in 5'-Position verknüpft. In einer besonders bevorzugten Ausführungsform ist die Gegenstrangnukleinsäure in dem Expressionsvektor in 3'-Position mit einem Reportergen und in 5'-Position mit einer Expressionskontrollsequenz operativ verknüpft.

Ein erfindungsgemäßer Expressionsvektor kann auf beliebige Weise erzeugt werden, beispielsweise durch Kultivierung in Wirtszellen, vorzugsweise in Bakterienzellen wie etwa *E. coli*-Zellen. Zweckmäßigerweise beinhaltet der Expressionsvektor Elemente, die eine Replikation und Selektion in der Wirtszelle ermöglichen. Alternativ kann ein Expressionsvektor auch *in vitro* durch Amplifikation in ausreichender Menge erzeugt werden, beispielsweise durch Polymerase-Kettenreaktion (PCR), Ligase-Kettenreaktion (LCR) oder Rolling-Circle-Amplifikation.

Als Wirtszellen können prokaryotische oder eukaryotische Zellen oder Mikroorganismen verwendet werden. Bei den Wirtszellen kann es sich um Wildtyp-Varianten handeln oder es können mutierte oder genetisch manipulierte Wirtszellen verwendet werden.

Als Reportergen kann gemäß der Erfindung ein für ein nachweisbares Genprodukt kodierendes Gen verwendet werden. Beispielsweise kann das Reportergen ein Antibiotika-Resistenzgen, vorzugsweise Kanamycin-Resistenz umfassen. In einer anderen Ausführungsform kann ein Reportergen verwendet werden, welches für ein Fluoreszenzprotein wie etwa GFP kodiert. Weiterhin kann ein Reportergen verwendet werden, das für ein Enzym kodiert, durch das eine colorimetrisch messbare Reaktion katalysiert wird (z.B. β-Galaktosidase). In einer weiteren Ausführungsform kann das Reportergen für ein Genprodukt kodieren, welches mit einer in der Wirtszelle vorhandenen Nukleinsäuresequenz oder mit einem in der Wirtszelle expremierten Genprodukt wechselwirkt, wodurch es zu einer messbaren Veränderung einer Stoffwechselleistung kommt.

Das Einbringen von Expressionsvektor und gegebenenfalls Reportervektor in die Wirtszelle erfolgt nach im Stand der Technik bekannten Verfahren, beispielsweise durch (Co)Transfektion, (Co)Transformation oder (Co)Infektion von Zellen. Vorzugsweise wird das Verfahren so gewählt, dass die doppelsträngige Nukleinsäure in die Wirtszelle eingebracht wird, sodass in der Wirtszelle die Gegenstrangsequenz operativ mit dem Reportergen verknüpft ist.

Bei eukaryotischen Wirtszellen kann die Transfektion bzw. Cotransfektion beispielsweise durch Calciumphosphat-Copräzipitation, Lipofektion, Elektroporation, Partikelbeschuss, Verwendung von Bakterienproteinen oder durch virale Infektion über Retroviren, Adenoviren etc. erfolgen.

In einer bevorzugten Ausführungsform der Erfindung wird in Schritt (i) des Verfahrens ein Vektor bereitgestellt, der zusätzlich zu dem operativ mit der Gegenstrangnukleinsäure verknüpften Reportergen mindestens ein Selektionsmarkergen umfasst. Das mindestens eine Selektionsmarkergen ist ausgewählt aus beliebigen für ein nachweisbares Genprodukt kodierenden Genen, wobei das Genprodukt vorzugsweise von dem Genprodukt des Reportergens verschieden ist. Beispielsweise kann das Selektionsmarkergen ein Antibiotikaresistenzgen wie etwa β-Lactamase für Ampicilinresistenz umfassen. Alternativ kann als Selektionsmarkergen ein für ein Fluoreszenzprotein wie etwa GFP kodierendes Gen verwendet werden.

Über das Selektionsmarkergen kann unabhängig von dem Reportergen eine Selektion erfolgen. Beispielsweise kann ein Konstrukt nach erfolgter Selektion auf das erste Reportergen (beispielsweise auf Kanamycinresistenz) über Selektion mittels des weiteren Selektionsmarkergens (z.B. für Ampicillinresistenz) weiter hochamplifiziert werden. Darüber hinaus besteht die Möglichkeit, Konstrukte die eine Frameshift-Mutation enthalten (z.B. eine absichtliche Frameshift-Mutation) durch Selektion über das zweite Selektionsmarkergen weiter zu amplifizieren.

Schließlich kann das zweite Selektionsmarkergen dazu beitragen, die Existenz des Reportergens im Zusammenhang mit einer stattgefundenen Selektion zu verschleiern, um so das Verfahren vor Nachahmung zu schützen.

Schritt (ii) des erfindungsgemäßen Verfahrens beinhaltet das Bewirken der Expression der Gegenstrangnukleinsäure in der Wirtszelle. Hierzu wird die Wirtszelle unter geeigneten Bedingungen kultiviert, die eine Expression der Gegenstrangnukleinsäure erlauben.

Schritt (iii) beinhaltet die Bestimmung, ob in der Wirtszelle eine Expression des Reportergens erfolgt. Die Bestimmung beruht darauf, dass ein von dem Reportervektor codiertes Genprodukt nachgewiesen wird. Die Bestimmung kann abhängig von dem verwendeten Reportergen grundsätzlich die Bestimmung beliebiger phänotypisch erkennbarer Effekte umfassen, beispielsweise morphologische Veränderungen, Änderungen des Wachstumsverhaltens, etc. Wenn das Reportergen beispielsweise ein Fluoreszenzprotein wie GFP kodiert, so kann das Vorhandensein und/oder die Intensität der Lumineszenz z.B. mittels Fluoreszenzcytometrie oder Imaging-Assays bestimmt werden. Wenn als Reportergen ein Antibiotika-Resistenzgen verwendet wird, so kann die Bestimmung auf Grundlage des Wachstums von Wirtszellen in Gegenwart von Antibiotika erfolgen.

In einer Ausführungsform der Erfindung kann eine Zielnukleinsäure aus zwei, drei oder mehreren Teilfragmenten zusammengesetzt sein. Beispielsweise können bei einem Konstrukt mit 3 kB zunächst drei Teilfragmente aus Oligonukleotiden hergestellt werden und diese Teilfragmente dann z.B. über Fusions-PCR zu einem 3 kB-Fragment fusioniert werden. Ein so fusioniertes Konstrukt aus zwei oder mehreren Teilfragmenten kann dann direkt in einen erfindungsgemäßen Expressionsvektor ligiert werden.

Da mit dem erfindungsgemäßen Verfahren Frameshift-Mutationen, aber keine Mutationen unter Erhalt des Leserasters bestimmt werden können, wird bei der Synthese der Zielnukleinsäure durch PCR vorzugsweise der Amplifikationsprimer zu einem möglichst späten Zeitpunkt zugegeben, um die Anzahl der Amplifikationsschritte möglichst klein zu halten. Je höher die Anzahl amplifizierbarer Volllängenmoleküle, desto weniger durch PCR zustandegekommene Mutationen sind im PCR-Produkt enthalten.

Um Substitutionen noch weiter zu verringern, kann beispielsweise Ofloxazin nach der Transformation eingesetzt werden. Ofloxazin ist ein Gyrasehemmer, der die DNA-Replikation hemmt, Wirtszellen wie *E. coli-*Zellen jedoch kurzfristig nicht tötet. Während dieses Zeitraums besteht für eine Wirtszelle die Möglichkeit, Heterodimere über einen internen Reparaturmechanismus zu spalten. Vorzugsweise kann Ofloxazin im Medium nach der Elektroporation oder Hitzeschocktransformation enthalten sein (0,5-1 h bei 37 °C). Im Anschluss werden die Zellen abzentrifugiert, das Medium zusammen mit dem Ofloxazin entfernt und die Zellen werden ausplattiert.

### Figuren

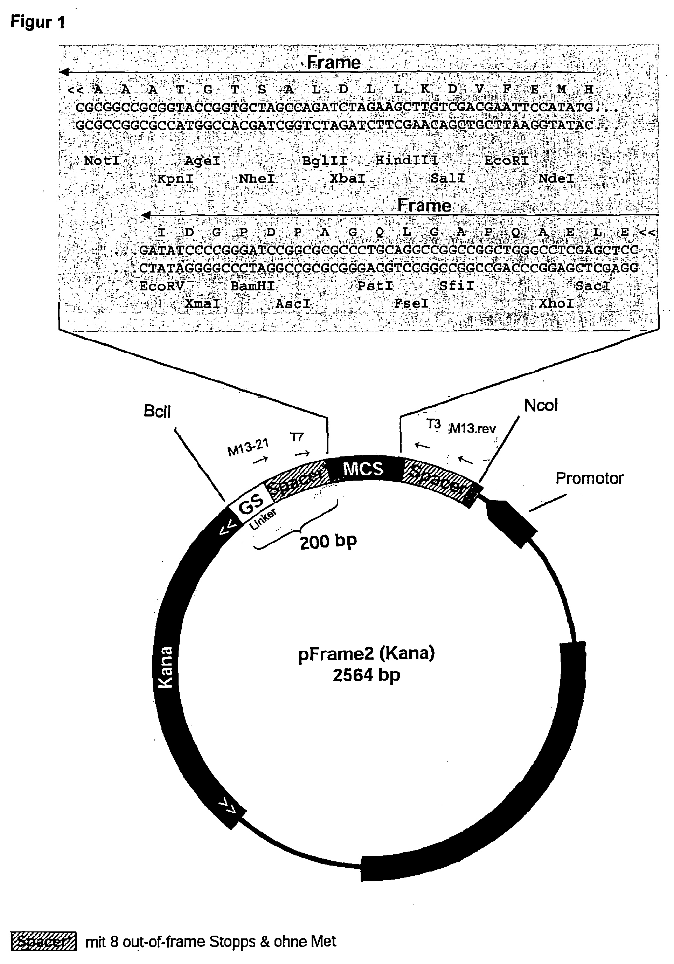

**Figur 1** zeigt die Plasmidkarte eines erfindungsgemäßen Expressionsvektors. Als Reportergen ist Kanamycinresistenz stromabwärts der Gegenstrangnukleinsäure MCS enthalten.

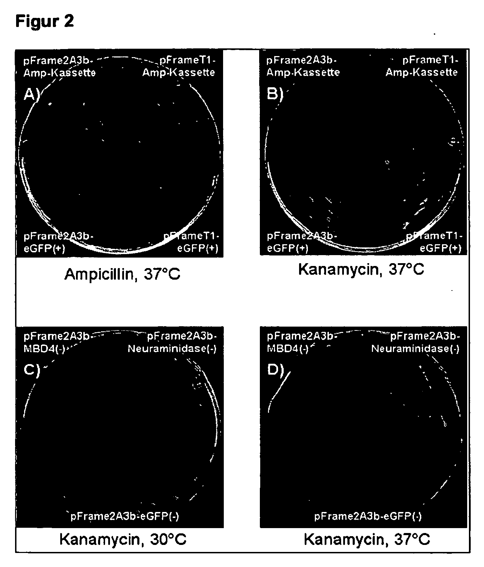

**Figur 2** Ausstrichplatten von Kolonien mit Kanamycin-Selektionsvektoren (pFrame2A3b und pFrameT1) mit unterschiedlichen Kpnl/Sacl Insertionen. Bei den Insertionen auf Platte A) und B) handelt es sich einerseits um beta-Lactamase inclusive Promotor (A und B, oben), welche in umgekehrter Orientierung zum Kanamycin-Resistenzgen kloniert vorliegt und Amp-Resistenz vermittelt, jedoch keine Kana-Resistenz zulässt, da der Gegenstrang von Promotor+beta-Lactamase keinen offenen Leserahmen beinhaltet, sowie andererseits um eGFP (A und B, unten), welches hier in gleicher Orientierung und in-frame zum Kanamycin-Resistenzgen kloniert vorliegt. Hier ermöglicht der durchgehende Leserahmen des Plusstranges von eGFP die Expression der Kanamycin-Resistenz, während die fehlende Ampicillin-Kassette natürlich kein Wachstum auf Ampicillin zulässt. Diese Daten zeigen, dass eine Selektion auf Sequenzen mit offenem Leserahmen mit Hilfe des erfindungsgemäßen Verfahrens möglich ist.
   Demgegenüber sind auf den Platten C) und D) drei Kolonien ausgestrichen, welche im Kanamycin-Selektionsvektor pFrame2A3b drei unterschiedliche Insertionen in negativer Orientierung beinhalten, wobei alle drei einen offenen Leserahmen auch im Minusstrang besitzen. Bei den Insertionen handelt es sich um die Gene für eGFP (735 bp), Influenza-Neuraminidase (1398 bp) und humanes CpG-bindendes Protein MBD4 (1755 bp).
   Alle drei Konstrukte sind dazu in der Lage, auf Kanamycin zu wachsen. Es ist jedoch auch deutlich, dass im Einzelfall - hier insbesondere MBD4 (-) und im geringerem Maße auch Neuraminidase(-) und eGFP(-) - das Wachstum bei 30 °C anstelle von 37 °C stabiler ist.
   Diese drei Fusionsproteine aus Minusstrang-Translation plus Neomycin-Phosphotransferase sind also dazu in der Lage, die phänotypische & selektierbare Kanamycin-Resistenz zu vermitteln.

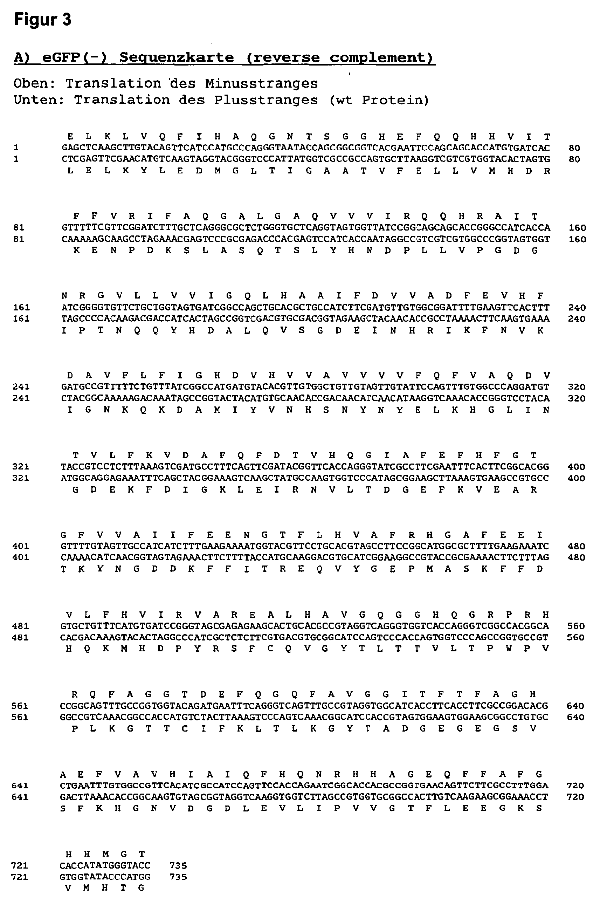

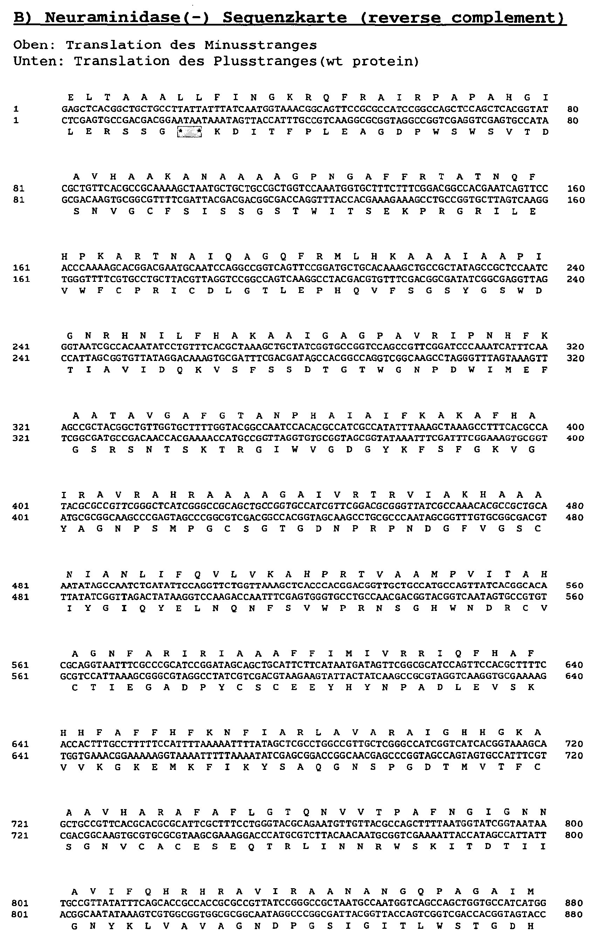

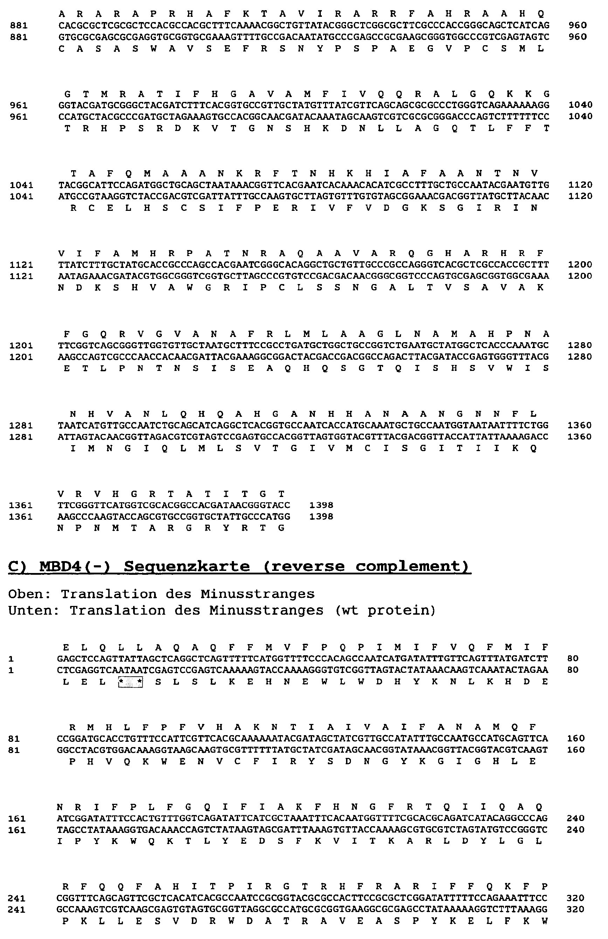

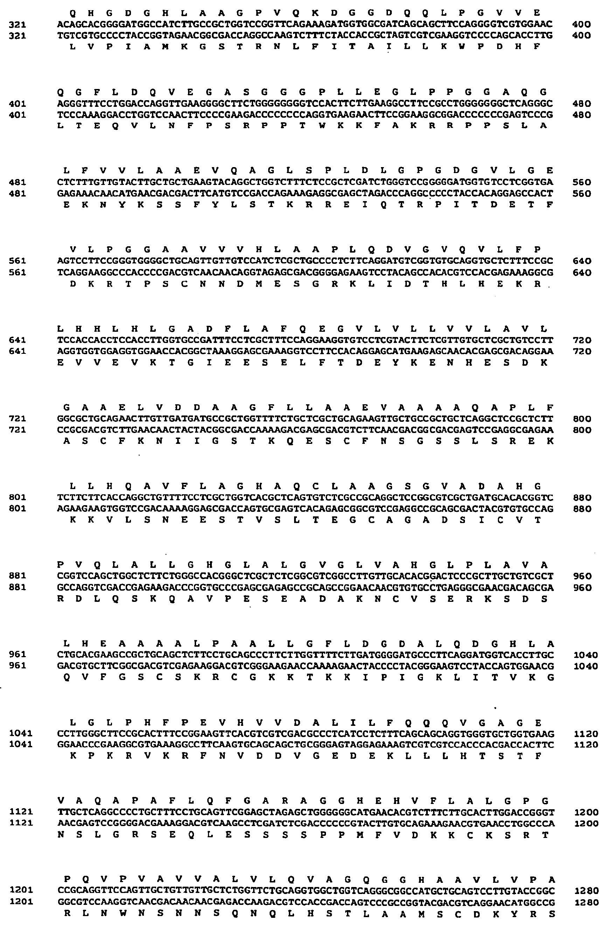

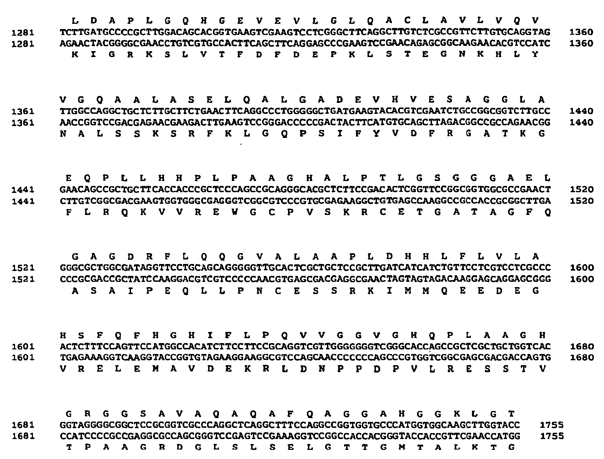

**Figur 3** zeigt die Sequenzen der drei Insertionen aus Fig. 2 mit annotierter Plus- und Minustranslation.
**Figur 4** veranschaulicht einen Vergleich der Selektion codierender Nukleinsäurekonstrukte auf Abwesenheit von Framshiftmutationen mit Hilfe eines klassischen Klonierungsvektors und eines erfindungsgemäßen Klonierungsvektors. Die grauen Balken zeigen das Ergebnis der Selektion unter Verwendung des klassischen Klonierungssektors pCR-Skript, die weißen Balken zeigen das Ergebnis der Selektion unter Verwendung des erfindungsgemäßen Selektionsvektors pFr2b.

### Beispiel

### Selektion auf Abwesenheit von Frameshiftmutationen

Ein komplett aus Oligonukleotiden hergestelltes Konstrukt mit einer Länge von 684 bp wurde über die Restriktionsenzyme Kpnl/Sacl in den herkömlichen Klonierungssektor pCR-Skript und in den erfindungsgemäßen Selektionsvektor pFr2b kloniert. Die Sequenz des Konstrukts wurde über alternative Codonwahl so konzipiert, dass sie neben dem biologisch relevanten Leserahmen (mit terminalem Stoppkodon) einen zweiten offenen Leserahmen in Gegenrichtung aufweist. Dies hat jedoch kein Einfluss auf das auf dem Sinnstrang kodierte biologisch relevante Protein.

Durch die Ligation des Konstrukts (Insert) in den Vektor pFr2b entsteht ein offener Leserahmen innerhalb des Vektors, der sich zusammensetzt aus Start + reverges Insert + Selektionsgen (Kana). Nur bei intaktem Leserahmen des Inserts kann Kana korrekt translatiert werden und der Zelle/ Kolonie das Wachstum ermöglichen.

Die Ligationen wurden in *E. coli* transformiert und auf Selektionsplatten mit 50 µ/ml Kanamycin ausplattiert. Von gewachsenen Einzelkolonien wurde Plasmid-DNA präpariert, sequenziert und nach den folgenden, in Tabelle 1 aufgeführten Kriterien analysiert.

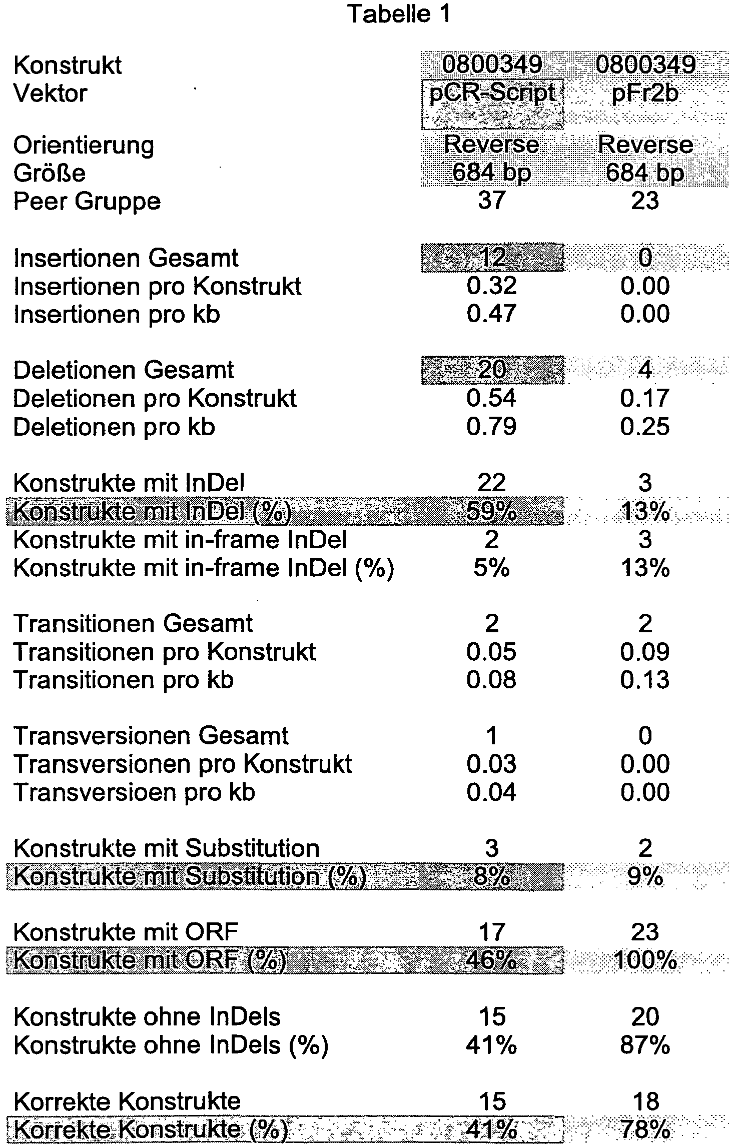

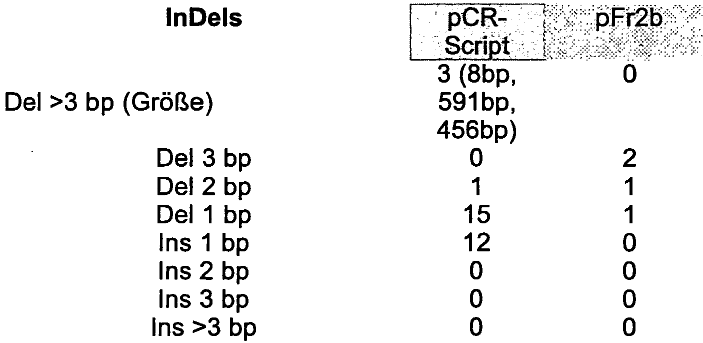

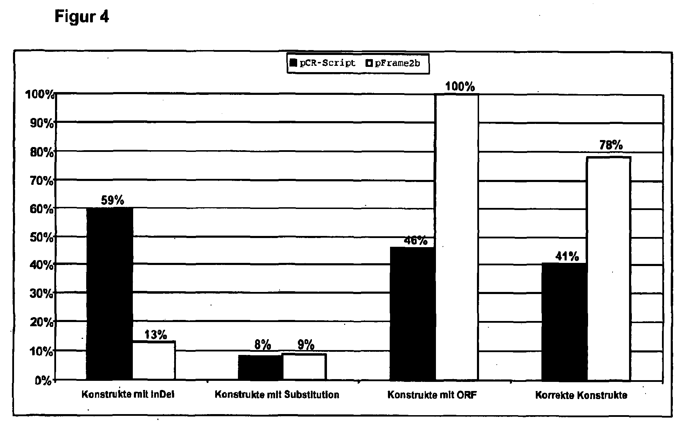

Die in der vorstehenden Tabelle 1 verwendeten Begriffe und Abkürzungen haben die nachstehenden Bedeutungen:
- **Konstrukt**: Bezeichnung des Konstrukts. Das Gen wurde jeweils über Kpnl/Sacl in die Vektoren kloniert.
- **Vektor**: pCR-Script: klassischer Klonierungsvektor; pFr2b: erfindungsgemäßer Selektionsvektor für offene Leserahmen
- **Orientierung**: Orientierung, in die in den Vektor kloniert wurde. Nur relevant für pFr2b. Hier wurde also so kloniert, dass der reverse Leserahmen des Gens mit der Selektionskassette fusioniert vorliegt.
- **Größe**: Länge des Gens in bp.
- **Peer Gruppe**: Anzahl der sequenzierten Klone.
- **Insertionen Gesamt**: Gesamtzahl der in der Stichprobe gefundenen Insertionen.
- **Insertionen pro Konstrukt**: Berechnete Insertionen pro Konstrukt.
- **Insertionen pro kb**: Berechnete Insertionen pro Kilobase.
- **Deletionen Gesamt**: Gesamtzahl der in der Stichprobe gefundenen Deletionen.
- **Deletionen pro Konstrukt**: Berechnete Deletionen pro Konstrukt.
- **Deletionen pro kb**: Berechnete Deletionen pro Kilobase.
- **Konstrukte mit InDel**: Gesamtzahl der in der Stichprobe gefundenen Konstrukte mit mindestens einer Insertion oder Deletion. Im Falle von pFr2b ist dies nicht identisch mit der Summe aller Insertionen + Deletionen, da es hier z.B ein Konstrukt mit zwei Deletionen gibt.
- **Konstrukte mit InDel (%)**: Prozentualer Anteil an Konstrukten in der Stichprobe mit mindestens einer Insertion oder Deletion. Im Falle von pFr2b werden deutlich weniger Konstrukte mit InDels gefunden (13% gegenüber 59%). Siehe Figur 4.
- **Konstrukte mit in-frame InDel**: Gesamtzahl der in der Stichprobe gefundenen Konstrukte mit mindestens einer Insertion oder Deletion, die jedoch nicht zu einer Unterbrechung des offenen reversen Leserahmens führen. Man beachte, dass im Falle von pFr2b die drei Konstrukte mit einer InDel noch immer einen intakten offenen reversen Leserahmen aufweisen. Dies liegt daran, dass zwei Konstrukte eine Deletion von 3 bp haben, ein Konstrukt eine Deletion von 1 bp und kurz dahinter noch einmal eine Deletion von 2 bp - die Leserahmen werden hier nicht unterbrochen, da die Deletionen durch drei teilbar sind, wie im allgemeinen Teil der Beschreibung erwähnt ist.
- **Konstrukte mit in-frame InDel**: **(%)** Prozentualer Anteil an Konstrukten in der Stichprobe, mit mindestens einer Insertion oder Deletion, die jedoch nicht zu einer Unterbrechung des offenen reversen Leserahmens führen.
- **Transitionen Gesamt**: Gesamtzahl der in der Stichprobe gefundenen Transitionen. (Purin <-> Purin oder Pyrimidin <-> Pyrimidin). Die Tatsache, dass Transitionen häufiger beobachtet werden als Transversionen, deutet darauf hin, dass die Substitutionen nicht durch Oligonukleotidfehler eingeführt wurden, sondern durch PCR-Fehler.
- **Transitionen pro Konstrukt**: Berechnete Transitionen pro Konstrukt.
- **Transitionen pro kb**: Berechnete Transitionen pro Kilobase.
- **Transversionen Gesamt**: Gesamtzahl der in der Stichprobe gefundenen Transversionen. (Purin <-> Pyrimidin).
- **Transversionen pro Konstrukt**: Berechnete Transversionen pro Konstrukt.
- **Transversionen pro kb**: Berechnete Transversionen pro Kilobase.
- **Konstrukte mit Substitution**: Gesamtzahl der in der Stichprobe gefundenen Transitionen + Transversionen (= Substitutionen)
- **Konstrukte mit Substitution (%)**: Prozentualer Anteil an Konstrukten in der Stichprobe, mit mindestens einer Substitution. Bezüglich der Substitutionen findet keine Selektion durch pFr2b statt. Siehe Figur 4.
- **Konstrukte mit ORF**: Gesamtzahl der in der Stichprobe gefundenen Konstrukte mit einem offenen reversen Leserahmen.
- **Konstrukte mit ORF (%)**: Prozentualer Anteil an Konstrukten in der Stichprobe mit einem offenen reversen Leserahmen. Man beachte, dass dieser Wert im Falle von pFr2b bei 100% liegt. Siehe Figur 2.
- **Konstrukte ohne InDels**: Gesamtzahl der in der Stichprobe gefundenen Konstrukte ohne eine Insertion oder Deletion.
- **Konstrukte ohne InDels (%)**: Prozentualer Anteil an Konstrukten in der Stichprobe ohne Insertion oder Deletion. Man beachte, dass dieser Wert im Falle von pFr2b bei 87% liegt, also mehr als doppelt so hoch ist wie ohne entsprechende Selektion.
- **Korrekte Konstrukte**: Gesamtzahl der in der Stichprobe gefundenen Konstrukte, die weder eine Insertion oder Deletion, noch eine Substitution haben - also zu 100% korrekt sind.
- **Korrekte Konstrukte (%)**: Prozentualer Anteil an Konstrukten in der Stichprobe die weder eine Insertion oder Deletion, noch eine Substitution haben - also zu 100% korrekt sind. Man beachte, dass dieser Wert bei im Falle von pFr2b bei 87% liegt, also fast doppelt so hoch ist wie ohne entsprechende Selektion. Siehe Figur 4.

**Del >3 (Größe)** Gesamtzahl der in der Stichprobe gefundenen Deletionen > 3 bp und deren Größe.
- **Del 3**: Gesamtzahl der in der Stichprobe gefundenen Deletionen von 3 bp.
- **Del 2**: Gesamtzahl der in der Stichprobe gefundenen Deletionen von 2 bp.
- **Del 1**: Gesamtzahl der in der Stichprobe gefundenen Deletionen von 1 bp.
- **Ins 1**: Gesamtzahl der in der Stichprobe gefundenen Insertionen von 1 bp.
- **Ins 2**: Gesamtzahl der in der Stichprobe gefundenen Insertionen von 2 bp.
- **Ins 3**: Gesamtzahl der in der Stichprobe gefundenen Insertionen von 3 bp.
- **Ins >3**: Gesamtzahl der in der Stichprobe gefundenen Deletionen > 3 bp.

### Ergebnis:

Die Klonierung in den Selektionsvektor pFr2b führt im gegebenen Beispiel zu einer 100 %igen Eliminierung von Inserts, deren Leserahmen durch Insertionen oder Deletionen zerstört ist. Eine Selektion auf die weitaus selteneren Substitutionsmutationen findet jedoch nicht statt. Auch Insertionen/Deletionen, die den Leserahmen intakt lassen, werden nicht eliminiert.

Insgesamt führte die Selektion im gegebenen Beispiel zu einer Ausbeute von 78 % korrekter Konstrukte, während ohne Selektion nur 41 % der Konstrukte korrekt waren. Das Ergebnis der Untersuchung der Selektion ist in Figur 2 veranschaulicht.

## Patentansprüche

1. Verfahren zur Bestimmung von Frameshift-Mutationen in kodierenden Zielnukleinsäuren, welches die Schritte umfasst:
(i) Bereitstellen einer Wirtszelle, umfassend eine doppelsträngige Nukleinsäure, welche eine kodierende Zielnukleinsäure und eine dazu komplementäre, kodierende Gegenstrangnukleinsäure umfasst, worin die Gegenstrangnukleinsäure in operativer Verknüpfung mit einem Reportergen in 3' Position vorliegt, und worin die Gegenstrangnukleinsäure so optimiert wird, dass sie keine Stopp-Kodons umfasst;
(ii) Bewirken der Expression der Gegenstrangnukleinsäure; und
(iii) Bestimmen, ob in der Wirtszelle eine Expression des Reportergens erfolgt, worin die Expression des Reportergens anzeigt, dass die Zielnukleinsäure keine Frameshift-Mutation aufweist,
wobei die Wirtszelle keine menschliche embryonale Zelle ist.

2. Verfahren nach Anspruch 1, worin die Gegenstrangnukleinsäure weiterhin in operativer Verknüpfung mit einer Expressionskontrollsequenz in 5' Position vorliegt.

3. Verfahren nach Anspruch 1 oder 2, worin Schritt (i) umfasst:
Einbringen eines Expressionsvektors, umfassend die doppelsträngige Nukleinsäure, welche eine kodierende Zielnukleinsäure und eine dazu komplementäre, kodierende Gegenstrangnukleinsäure umfasst, in eine Wirtszelle.

4. Verfahren nach Anspruch 3, worin die Gegenstrangnukleinsäure in dem Expressionsvektor in operativer Verknüpfung mit einem Reportergen in 3' Position und/oder mit einer Expressionskontrollsequenz in 5' Position vorliegt.

5. Verfahren nach Anspruch 3, worin der Expressionsvektor zusammen mit einem Reportervektor, welcher das Reportergen umfasst, in die Wirtszelle eingebracht wird.

6. Verfahren nach Anspruch 3, worin der Expressionsvektor in eine Wirtszelle eingebracht wird, die bereits ein Reportergen enthält.

7. Verfahren nach einem der Ansprüche 3 bis 6, worin in Schritt (i) ein Expressionsvektor verwendet wird, der zusätzlich mindestens ein Selektionsmarkergen umfasst, das bevorzugt konstitutiv exprimiert wird.

8. Verfahren nach einem der vorhergehenden Ansprüche, worin die Nukleinsäuresequenz aus genomischen Sequenzen, cDNA-Sequenzen, cDNA-Fragmenten oder aus einer Bibliothek, die eine Kollektion von Sequenzen enthält, stammt.

9. Verfahren nach einem der vorhergehenden Ansprüche, worin die Zielnukleinsäuresequenz aus einer normalisierten cDNA-Bibliothek stammt.

10. Verfahren nach einem der vorhergehenden Ansprüche, worin als Expressionsvektor ein Plasmid verwendet wird.

11. Verfahren nach einem der vorhergehenden Ansprüche, worin als Wirtszelle eine eukaryotische Zelle verwendet wird.

12. Verfahren nach einem der Ansprüche 3 bis 11, worin das Einbringen des Expressionsvektors durch Calciumphosphat-Copräzipitation, Lipofektion, Elektroporation, Partikelbeschuss oder virale Infektion erfolgt.

13. Verfahren nach einem der vorhergehenden Ansprüche, worin das Reportergen ein für ein nachweisbares Genprodukt codierendes Gen enthält.

14. Verfahren nach Anspruch 13, worin das Reportergen ein Antibiotika-Resistenzgen wie etwa Kanamycin-Resistenz umfasst, oder das nachweisbare Genprodukt aus Fluoreszenzproteinen wie vorzugsweise GFP ausgewählt wird oder eine colorimetrisch messbare Reaktion katalysiert.

15. Verfahren nach einem der vorhergehenden Ansprüche, worin die Bestimmung in Schritt (iii) durch Fluoreszenzcytometrie oder Imaging-Assays erfolgt.

## Claims

1. A method for determining frameshift mutations in coding target nucleic acids, with the following steps:
(i) providing a host cell comprising a double-stranded nucleic acid which includes a coding nucleic acid and a coding reverse strand nucleic acid complementary thereto, wherein the reverse strand nucleic acid has an operative linkage in the 3' position to a reporter gene, and wherein the reverse strand nucleic acid is optimized in such a manner that it does not contain any stop codons;
(ii) implementing the expression of the reverse strand nucleic acid; and
(iii) determining whether the reporter gene is expressed in the host cell, wherein the expression of the reporter gene indicates that the target nucleic acid has no frameshift mutation,
wherein the host cell is not a human embryonic cell.

2. The method according to claim 1, wherein the reverse strand nucleic acid furthermore has an operative linkage in the 5' position to an expression control sequence.

3. The method according to claim 1 or 2, wherein step (i) includes:
inserting an expression vector, comprising the double-stranded nucleic acid, the same having a coding target nucleic acid and a coding reverse strand nucleic acid complementary thereto, into a host cell.

4. The method according to claim 3, wherein the reverse strand nucleic acid in the expression vector has an operative linkage in the 3' position to a reporter gene and/or in the 5' position to an expression control sequence.

5. The method according to claim 3, wherein the expression vector is inserted into the host cell together with a reporter vector which includes the reporter gene.

6. The method according to claim 3, wherein the expression vector is inserted into a host cell which already contains a reporter gene.

7. The method according to one of the claims 3 to 6, wherein in step (i), an expression vector is used which additionally contains at least one selection marker gene, which is preferably expressed constitutively.

8. The method according to one of the previous claims, wherein the nucleic acid sequence originates from genomic sequences, cDNA sequences, cDNA fragments, or from a library which contains a collection of sequences.

9. The method according to one of the previous claims, wherein the target nucleic acid sequence originates from a normalized cDNA library.

10. The method according to one of the previous claims, wherein a plasmid is used as the expression vector.

11. The method according to one of the previous claims, wherein a eukaryotic cell is used as the host cell.

12. The method according to one of the claims 3 to 11, wherein the expression vector is inserted by calcium phosphate co-precipitation, lipofection, electroporation, particle bombardment, or viral infection.

13. The method according to one of the previous claims, wherein the reporter gene contains a gene coding for a detectable gene product.

14. The method according to claim 13, wherein the reporter gene includes an antibiotic resistance gene such as kanamycin resistance, or the detectable gene product is selected from among fluorescence proteins such as, preferably, GFP, or catalyzes a reaction measurable by colorimetry.

15. The method according to one of the previous claims, wherein the determination in step (iii) is made using fluorescence cytometry or imaging assays.

## Revendications

1. Procédé de détermination de mutations frameshift dans des acides nucléiques cibles codants, ledit procédé comprenant les étapes suivantes :
i) préparation d'une cellule hôte, incluant un acide nucléique bicaténaire, qui inclut une acide nucléique cible codant et un acide nucléique antisens codant complémentaire, l'acide nucléique antisens étant présent en relation opérationnelle avec un gène rapporteur en position 3', et l'acide nucléique antisens étant optimisé de façon à ne comprendre aucun codon stop ;
ii) déclenchement de l'expression de l'acide nucléique antisens ; et
iii) détermination de si une expression du gène rapporteur a lieu dans la cellule hôte, l'expression du gène rapporteur indiquant que l'acide nucléique cible ne présente aucune mutation frameshift,
la cellule hôte n'étant pas une cellule embryonnaire humaine.

2. Procédé selon la revendication 1, dans lequel l'acide nucléique antisens est en relation opérationnelle avec une séquence de contrôle d'expression en position 5'.

3. Procédé selon la revendication 1 ou 2, dans lequel l'étape i) comprend l'introduction d'un vecteur d'expression, incluant un acide nucléique bicaténaire comprenant un acide nucléique cible codant et un acide nucléique antisens codant complémentaire, dans une cellule hôte.

4. Procédé selon la revendication 3, dans lequel l'acide nucléique antisens dans le vecteur d'expression est en relation opérationnelle avec un gène rapporteur en position 3' et/ou avec une séquence de contrôle d'expression 5'.

5. Procédé selon la revendication 3, dans lequel le vecteur d'expression est introduit dans la cellule hôte associé à un vecteur rapporteur qui comprend le gène rapporteur.

6. Procédé selon la revendication 3, dans lequel le vecteur d'expression est introduit dans une cellule hôte qui contient déjà un gène rapporteur.

7. Procédé selon l'une des revendications 3 à 6 dans lequel, à l'étape i), on utilise un vecteur d'expression qui comprend en plus au moins un gène marqueur de sélection qui est préférentiellement exprimé constitutivement.

8. Procédé selon l'une des revendications précédentes, dans lequel la séquence d'acide nucléique provient de séquences génomiques, de séquences de cADN, de fragments de cADN ou d'une bibliothèque contenant une collection de séquences.

9. Procédé selon l'une des revendications précédentes, dans lequel la séquence d'acide nucléique cible provient d'une bibliothèque de cADN normalisée.

10. Procédé selon l'une des revendications précédentes, dans lequel on utilise un plasmide comme vecteur d'expression.

11. Procédé selon l'une des revendications précédentes, dans lequel on utilise comme cellule hôte une cellule eucaryote.

12. Procédé selon l'une des revendications 3 à 11 dans lequel l'introduction du vecteur d'expression se fait par coprécipitation au phosphate de calcium, lipofection, électroporation, bombardement de particules ou infection virale.

13. Procédé selon l'une des revendications précédentes, dans lequel le gène rapporteur contient un gène codant un produit génique détectable.

14. Procédé selon la revendication 13, dans lequel le gène rapporteur contient un gène de résistance à un antibiotique, comme par exemple de résistance à la kanamycine, ou le produit génique détectable est choisi parmi des protéines fluorescentes, comme par exemple la GFP, ou catalyse une réaction mesurable par colorimétrie.

15. Procédé selon l'une des revendications précédentes, dans lequel la détermination à l'étape iii) se fait par cytométrie de flux à fluorescence ou par essais d'imagerie.
